# EUROPÄISCHE PATENTSCHRIFT

(11) **EP 1 592 696 B1**
(45) Veröffentlichungstag und Bekanntmachung des Hinweises auf die Patenterteilung: **27.09.2006**
(21) Anmeldenummer: 04702317.1
(22) Anmeldetag: 15.01.2004
(51) Int. Cl.: C07F 5/02

(54) **SALZE MIT CYANOBORAT-ANIONEN**
SALTS COMPRISING CYANOBORATE ANIONS
SELS A BASE D'ANIONS CYANOBORATES

(30) Priorität: 14.02.2003 DE 10306617
(43) Veröffentlichungstag der Anmeldung: 09.11.2005
(60) Teilanmeldung: 06016494.4
(73) Patentinhaber: Merck Patent GmbH, 64293 Darmstadt (DE)
(72) Erfinder: WELZ-BIERMANN, Urs, 64646 Heppenheim (DE); IGNATYEV, Nikolai, 47058 Duisburg (DE); BERNHARDT, Eduard, 42107 Wuppertal (DE); FINZE, Maik, 31582 Nienburg (DE); WILLNER, Helge, 45481 Muelheim/Ruhr (DE)
(86) Internationale Anmeldenummer: PCT/EP2004/000231
(87) Internationale Veröffentlichungsnummer: WO 2004/072089

(56) Entgegenhaltungen:
- WILLIAMS, DARRICK ET AL: "Synthesis of LiBC4N4, BC3N3, and Related C-N Compounds of Boron: New Precursors to Light Element Ceramics" JOURNAL OF THE AMERICAN CHEMICAL SOCIETY , 122(32), 7735-7741 CODEN: JACSAT; ISSN: 0002-7863, 2000, XP002278152 in der Anmeldung erwähnt
- BERNHARDT, E. ET AL: "The tetracyanoborates M[B(CN)4], M = [Bu4N]+, Ag+, K+" ZEITSCHRIFT FUER ANORGANISCHE UND ALLGEMEINE CHEMIE , 626(2), 560-568 CODEN: ZAACAB; ISSN: 0044-2313, 2000, XP0009030010 in der Anmeldung erwähnt
- BERNHARDT, E. ET AL: "An efficient synthesis for tetracyanoborates by sinter processes" ZEITSCHRIFT FUER ANORGANISCHE UND ALLGEMEINE CHEMIE , 629(7-8), 1229-1234 CODEN: ZAACAB; ISSN: 0044-2313, 2003, XP0009030008

## Beschreibung

Die vorliegende Erfindung betrifft ein Verfahren zur Herstellung von Salzen aus Cyanoborat-Anionen und organischen Kationen, diese Salze sowie ihre Verwendung als ionische Flüssigkeiten.

Ionische Flüssigkeiten oder flüssige Salze sind ionische Spezies, die aus einem organischen Kation und einem i.d.R. anorganischen Anion bestehen. Sie enthalten keine neutralen Moleküle, und weisen in der Regel Schmelzpunkte kleiner 373 K auf. Im Stand der Technik sind eine Vielzahl von Verbindungen bekannt, die als ionische Flüssigkeiten Verwendung finden. Insbesondere sind sie auch Gegenstand einer Reihe von Patenten bzw. Patentanmeldungen.

So wurden lösungsmittelfreie ionische Flüssigkeiten erstmals von Hurley und Wier in einer Reihe von US-Patenten (US 2,446,331, US 2,446,339 und US 2,446,350) offenbart. Diese "bei Raumtemperatur geschmolzenen Salze" enthielten AlCl₃ und eine Vielzahl von n-Alkylpyridinium-Halogeniden.

In den letzten Jahren wurden einige Übersichtsartikel zu diesem Thema veröffentlicht (R. Sheldon "Catalytic reactions in ionic liquids", *Chem. Commun.,* 2001, 2399-2407; M.J. Earle, K.R. Seddon "Ionic liquids. Green solvent for the future", *Pure Appl. Chem.,* 72 (2000), 1391-1398; P. Wasserscheid, W. Keim "Ionische Flüssigkeiten - neue Lösungen für die Übergangsmetallkatalyse", *Angew. Chem.,* 112 (2000), 3926-3945; T. Welton "Room temperature ionic liquids. Solvents for synthesis and catalysis", *Chem. Rev.,* 92 (1999), 2071-2083; R. Hagiwara, Ya. Ito "Room temperature ionic liquids of alkylimidazolium cations and fluoroanions", *Journal of Fluorine Chem.,* 105 (2000), 221-227).

Die Eigenschaften ionischer Flüssigkeiten, z.B. Schmelzpunkt, thermische und elektrochemische Stabilität, Viskosität, werden stark von der Natur des Anions beeinflusst. Demgegenüber können die Polarität und die Hydrophilie bzw. Lipophilie durch die geeignete Wahl des Kation/Anion-Paares variiert werden. Daher besteht grundsätzlicher Bedarf an neuen ionischen Flüssigkeiten mit variierten Eigenschaften, die zusätzliche Möglichkeiten hinsichtlich ihrer Verwendung ermöglichen.

Entscheidende Fortschritte auf dem Gebiet der ionischen Flüssigkeiten wurden mit der Entdeckung des 1-Ethyl-3-methylimidazolium Chloroaluminats erreicht. Dieses Salz hat einen breiten Flüssigbereich und ein elektrochemisches Fenster von mehr als 3 V und ist somit von großem Interesse für elektrochemische und synthetische Zwecke. Seine Anwendung ist aber durch die chemische Instabilität, vor allem gegen Feuchtigkeit, begrenzt. Nach der Entdeckung des hydrolysestabileren 1-Ethyl-3-methylimidazolium Tetrafluorborats wurden Kombinationen von Alkylimidazolium-Kationen mit anorganischen oder organischen Anionen untersucht, von denen das 1-Ethyl-3-methylimidazolium Tetrafluorborat am besten charakterisiert ist.

Die Stabilität des lmidazolium-Kations ist relativ hoch und seine Zersetzungstemperatur wird im wesentlichen durch das Anion bestimmt. So sind die 1-Ethyl-3-methylimidazolium-Salze mit Triflat- und Bis(trifluormethylsulfonyl)imid-Anionen bis 400°C stabil, wohingegen das 1-Ethyl-3-methylimidazolium Tetrafluorborat nur bis 300°C stabil ist.

Im Stand der Technik sind Borat-Anionen beschrieben, bei denen Fluor-Liganden durch Cyanid (E. Bernhardt, G. Henkel, H. Willner, *Z*. *Anorg. Allg. Chem.* 626 (2000) 560; D. Williams, B. Pleune, J. Kouvetakis, M. D. Williams, R. A. Andersen, *J. Amer. Chem. Soc.* 122 (2000) 7735; E. Bemhardt, M. Berkei, M. Schürmann, H. Willner, Z. *Anorg. Allg. Chem.* 628 (2002) 1734) und Trifluormethyl-Liganden (E. Bemhardt, G. Henkel, H. Willner, G. Pawelke, H. Bürger, *Chem. Eur. J.* 7 (2001) 4696; G. Pawelke, H. Bürger, *Coord. Chem. Rev.* 215 (2001) 243) ausgetauscht sind. Dabei werden die Trifluormethyl-Borate ausgehend von den Cyanoboraten synthetisiert, wobei allerdings die Cyanoborate schwer und nur in geringen Mengen zugänglich sind. Die Synthese von [B(CN₄)]⁻ ist arbeitsintensiv und nur in kleinem präparativem Maßstab durchführbar. Darüber hinaus sind die Ausgangsmaterialien teuer.

Aufgabe der vorliegenden Erfindung ist es, neue stabile Verbindungen mit wertvollen Eigenschaften, die als ionische Flüssigkeiten verwendet werden können, sowie ein Verfahren zu ihrer Herstellung zur Verfügung zu stellen. Insbesondere ist es Aufgabe, Salze mit Borat-Anionen zur Verfügung zu stellen, die eine höhere Stabilität haben als die Salze mit Tetrafluoroborat-Anionen.

Ferner ist es Aufgabe der vorliegenden Erfindung, ein effektives und wirtschaftlich sinnvolles Verfahren zur Herstellung dieser Borat-Salze und ihrer Vorläufer zur Verfügung zu stellen.

Diese Aufgabe wird erfindungsgemäß durch die kennzeichnenden Merkmale des Hauptanspruchs und der nebengeordneten Ansprüche gelöst.

Ein Gegenstand der vorliegenden Erfindung sind Verfahren zur Herstellung von Salzen mit Cyanoborat-Anionen der allgemeinen Formel (3) sowie die entsprechenden Salze der allgemeinen Formel (3)

Kt⁺ [BFₙ(CN)₄₋ₙ]⁻ (3),

wobei n = 0, 1, 2 oder 3 und Kt⁺ ein organisches Kation ist, mit der Maßgabe, dass für n=0 das Kation Kt⁺ nicht [N(C₄H₉)₄]⁺ ist.

Zur Herstellung der Salze wird ein Alkalimetallcyanoborat der allgemeinen Formel M⁺ [B(CN)₄]⁻, wobei M ausgewählt ist aus der Gruppe Li, Na, K, Rb und Cs, oder ein Alkalimetallcyanoborat der allgemeinen Formel M⁺ [BFₙ(CN)₄₋ₙ]⁻, wobei n = 0, 1, 2 oder 3 und M ausgewählt ist aus der Gruppe Li, Na, K, Rb und Cs,
mit Kt⁺ X⁻ umgesetzt,
wobei X ein Halogen ausgewählt aus CI, Br und I und Kt⁺ ein organisches Kation ist, mit der Maßgabe, dass das Kation Kt⁺ für n=0 nicht [N(C₄H₉)₄]⁺ ist.

Bevorzugt ist das organische Kation Kt⁺ ausgewählt aus der Gruppe 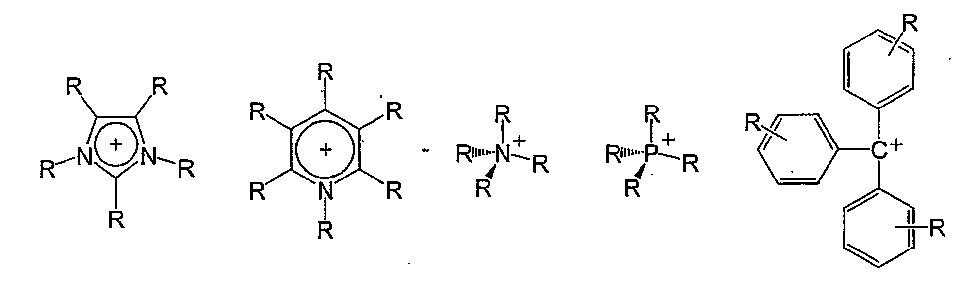 mit
- R =: H mit der Maßgabe, dass mindestens ein R am Heteroatom von H verschieden ist,
geradkettiges oder verzweigtes Alkyl mit 1-20 Kohlenstoffatomen
geradkettiges oder verzweigtes Alkenyl mit 2-20 Kohlenstoffatomen und einer oder mehreren Doppelbindungen
geradkettiges oder verzweigtes Alkinyl mit 2-20 Kohlenstoffatomen und einer oder mehreren Dreifachbindungen
gesättigtes, teilweise oder vollständig ungesättigtes Cycloalkyl mit 3-7 Kohlenstoffatomen
Halogen, insbesondere Fluor oder Chlor, mit der Maßgabe, dass keine Halogen-Heteroatom-Bindung vorliegt,
-NO₂ mit der Maßgabe, dass keine Bindung zu einem positiv geladenen Heteroatom vorliegt und mindestens ein R von NO₂ verschieden ist,
-CN mit der Maßgabe, dass keine Bindung zu einem positiv geladenen Heteroatom vorliegt und mindestens ein R von CN verschieden ist,
wobei die R jeweils gleich oder verschieden sind,
wobei die R paarweise durch Einfach- oder Doppelbindung miteinander verbunden sein können,
wobei ein oder mehrere R teilweise oder vollständig mit Halogenen, insbesondere -F und/oder -Cl, oder teilweise mit -CN oder -NO₂, substituiert sein können, mit der Maßgabe, dass nicht alle R vollständig halogeniert sind,
und wobei ein oder zwei Kohlenstoffatome des R durch Heteroatome und/oder Atomgruppierungen ausgewählt aus der Gruppe -O-, -C(O)-, C(O)O-, -S-, -S(O)-, -SO₂-, -S(O)₂O-, -N=, -P=, -NR'-, -PR'-, -P(O)(OR')-, -P(O)(OR')O-, -P(O)(NR'R')-, -P(O)(NR'R')O-, -P(O)(NR'R')NR'-, -S(O)NR'- und -S(O)₂NR'- mit R' = H, nicht, teilweise oder perfluoriertes C₁- bis C₆-Alkyl oder nicht, teilweise oder perfluoriertes Phenyl,
ersetzt sein können.

Unter vollständig ungesättigten Substituenten werden im Sinne der vorliegenden Erfindung auch aromatische Substituenten verstanden.

Als Substituenten R des organischen Kations kommen erfindungsgemäß dabei neben Wasserstoff in Frage: C₁- bis C₂₀-, insbesondere C₁- bis C₁₂-Alkylgruppen, C₂- bis C₂₀-, insbesondere C₂- bis C₁₂-, Alkenyl- oder Alkinylgruppen, gesättigte oder ungesättigte, d.h. auch aromatische, C₃- bis C₇-Cycloalkylgruppen, NO₂, CN oder Halogene. Für die Halogene gilt dabei allerdings einschränkend, dass sie nur als Substituenten an Kohlenstoffatomen, nicht aber an Heteroatomen auftreten. NO₂ und CN treten nicht als Substituenten eines positiv geladenen Heteroatoms auf; des Weiteren haben nicht alle Substituenten gleichzeitig die Bedeutung von NO₂ bzw. CN.

Die Substituenten R können auch paarweise derart verbunden sein, dass cyclische, bi- oder polycyclische Kationen entstehen. Die Substituenten können teilweise oder vollständig mit Halogenatomen, insbesondere mit F und/oder Cl, oder teilweise mit CN oder NO₂ substituiert sein und ein oder zwei Heteroatome oder Atomgruppierungen, ausgewählt aus der Gruppe O, C(O),C(O)O, S, S(O), SO₂, SO₂O, N, P, NH, PH, NR', PR', P(O)(OR'), P(O)(OR')O, P(O)(NR'R'), P(O)(NR'R')O, P(O)(NR'R')NR', S(O)NR' und S(O)₂NR' enthalten. In Fall einer vollständigen Halogenierung dürfen jedoch nicht alle vorhandenen Substituenten R vollständig halogeniert sein, d.h. mindestens ein R ist nicht perhalogeniert.

Ohne Einschränkung der Allgemeinheit sind Beispiele für erfindungsgemäße Substituenten des organischen Kations:
-F, -Cl, -Br, -I, -CH₃, -C₂H₅, -C₃H₇, -CH(CH₃)₂, -C₄H₉, -C(CH₃)₃, -C₅H₁₁, -C₆H₁₃, -C₆H₁₃, -C₇H₁₅, -C₈H₁₇, -C₉H₁₉, -C₁₀H₂₁, -C₁₂H₂₅, -C₂₀H₄₁, -OCH₃, -OCH(CH₃)₂, -CH₂OCH₃, -C₂H₄OCH(CH₃)₂, -SCH₃, -SCH(CH₃)₂, -C₂H₄SC₂H₅, -C₂H₄SCH(CH₃)₂, -S(O)CH₃, -SO₂CH₃, -SO₂C₂H₅, -SO₂C₃H₇, -SO₂CH(CH₃)₂, -CH₂SO₂CH₃, -OSO₂CH₃, -OSO₂CF₃, -CH₂N(H)C₂H₅, -C₂H₄N(H)C₂H₅, -CH₂N(CH₃)CH₃, -C₂H₄N(CH₃)CH₃, -N(CH₃)₂, -N(CH₃)C₃H₅, --N(CH₃)CF₃, O-C₄H₈-O-C₄H₉, -S-C₂H₄-N(C₄H₉)₂, -OCF₃, -S(O)CF₃, -SO₂CF₃, -CF₃, -C₂F₅, -C₃F₇, -C₄F₉, -C(CF₃)₃, -CF₂SO₂CF₃, -C₂F₄N(C₂F₅)C₂F₅, -CF=CF₂, -C(CF₃)=CFCF₃, -CF₂CF=CFCF₃, -CF=CFN(CF₃)CF₃, -CFH₂, -CHF₂, -CH₂CF₃, -C₂F₂H₃, -C₃FH₆, -CH₂C₃F₇, -C(CFH₂)₃, -CHO, -C(O)OH, -CH₂C(O)OH, -CH₂C(O)CH₃, -CH₂C(O)C₂H₅, -CH₂C(O)OCH₃, CH₂C(O)OC₂H₅, -C(O)CH₃, -C(O)OCH₃,

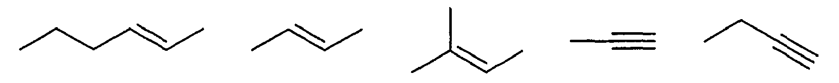

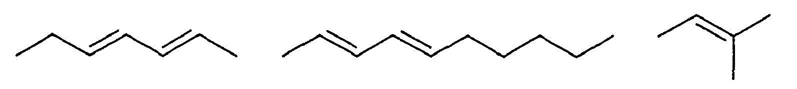

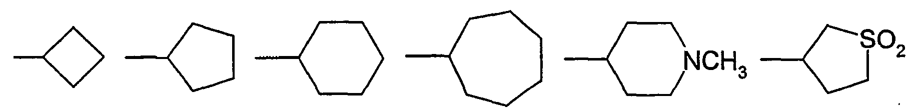

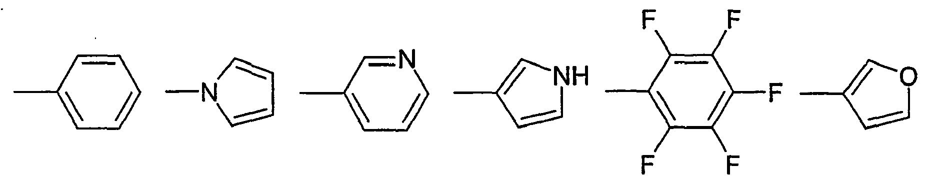

Ohne Einschränkung der Allgemeinheit sind die folgenden organische Kationen als erfindungsgemäße Salze besonders bevorzugt: 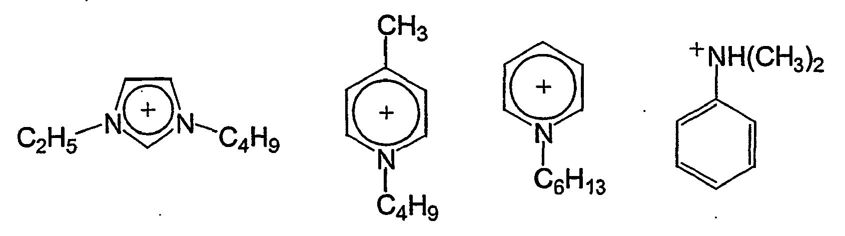 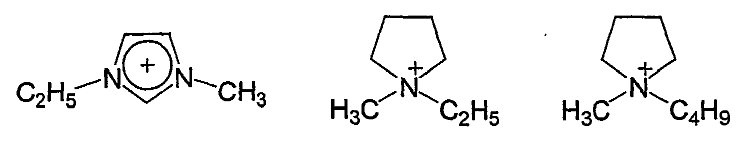 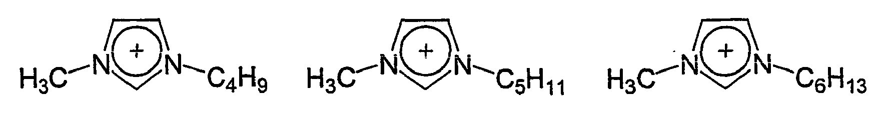 N(C₂H₅)₄⁺ N(C₄H₉)₄⁺ P(C₂H₅)₄⁺ P(C₄H₉)₄⁺ P(C₆H₁₃)₃(C₁₄H₂₉)⁺

Die erfindungsgemäßen Salze sind vorteilhafterweise sehr gut in organischen Lösungsmitteln löslich. Im Vergleich zu bekannten flüssigen Salzen besitzen die erfindungsgemäßen Salze überraschenderweise eine niedrige Viskosität. Vorteilhafterweise sind die erfindungsgemäßen Salze stabil. Sie können bei Raumtemperatur isoliert und gelagert werden. Ferner sind die erfindungsgemäßen Salze relativ leicht herstellbar und es werden leicht zugängliche Ausgangsmaterialien benötigt.

Alle erfindungsgemäßen Verbindungen sowie Verbindungen der Formel [N(C₄H₉)₄]⁺ [B(CN)₄]⁻ besitzen salzartigen Charakter, relativ niedrige Schmelzpunkte (meist unter 100°C) und können als ionische Flüssigkeiten verwendet werden.

Die erfindungsgemäßen Salze sowie Salze der Formel [N(C₄H₉)₄]⁺ [B(CN)₄]⁻ können als Lösungsmittel für viele synthetische oder katalytische Reaktionen eingesetzt werden, z.B. Friedel-Crafts-Acylierung und -Alkylierung, Diels-Alder-Cycloadditionen, Hydrogenierungs- und Oxidationsreaktionen, Heck-Reaktionen. Weiterhin können zum Beispiel fluorierte Lösungsmittel für sekundäre und primäre Batterien synthetisiert werden.

Die erfindungsgemäßen Salze sowie Salze der Formel [N(C₄H₉)₄]⁺ [B(CN)₄]⁻ eignen sich als Vorstufen für die Herstellung von Flüssigkristallverbindungen und von Wirkstoffen u.a. für Arznei- und Pflanzenschutzmittel.

Auch der Einsatz der erfindungsgemäßen Verbindungen sowie der Salze der Formel [N(C₄H₉)₄]⁺ [B(CN)₄]⁻ als nichtwässriger Elektrolyt gegebenenfalls in Kombination mit anderen, dem Fachmann bekannten Elektrolyten ist möglich.

Daneben sind die erfindungsgemäßen Salze sowie Salze der Formel [N(C₄H₉)₄]⁺ [B(CN)₄]⁻ als nichtwässerige, polare Substanzen in geeigneten Reaktionen als Phasentransferkatalysator oder als Medium zur Heterogenisierung von homogenen Katalysatoren von Interesse.

Die vollständige Offenbarung aller vor- und nachstehend aufgeführten Anmeldungen, Patente und Veröffentlichungen sind durch Bezugnahme in diese Anmeldung eingeführt.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die NMR-Spektren wurden an Lösungen in deuterierten Lösungsmitteln bei 20°C an einem Bruker Avance DRX-300 Spektrometer mit einem 5 mm Breitbandkopf ¹H/BB mit Deuterium Lock gemessen. Die Messfrequenzen der verschiedenen Kerne sind: ¹H: 300,13 MHz, ¹¹B: 96,92 MHz, ¹³C: 75,47 MHz, ¹⁹F: 282,41 MHz und ¹⁵N: 30,41 MHz. Die Methode der Referenzierung wird bei jedem Spektrum bzw. bei jedem Datensatz separat angegeben.

DSC-Messungen wurden an einem Netzsch DSC 204 Gerät durchgeführt. Zur Kalibrierung der Temperatur und der Empfindlichkeit fanden Naphthalin, Benzoesäure, KNO₃, AgNO₃, LiNO₃ und CsCI Verwendung. Von den Substanzen wurden jeweils 5-20 mg in einen Aluminium-Tiegel eingewogen und mit Aluminium-Kappen mit einer kleinen Öffnung verschlossen. Die Untersuchung erfolgte im Temperaturbereich von 25 bis 500°C. Sofern nicht anders angegeben beträgt die Aufheizrate 10 Kmin⁻¹. Während der Messung wurde der Probenraum mit trockenem Stickstoff gespült. Die Proben luftempfindlicher Substanzen wurden in einer Trockenbox präpariert und in einem mit Argon gefüllten Gläschen zum Analysengerät transportiert. Die Datenauswertung wurde mit dem Programm Netzsch Protens 4.0 vorgenommen.

Die Elementaranalysen erfolgten nach den Verbrennungsmethoden der Mikroanalytik mit einem Euro EA3000 der Firma HEKA-Tech GmbH. Die Proben luftempfindlicher Substanzen wurden in einer Trockenbox präpariert und in einem mit Argon gefüllten Gläschen zum Analysengerät transportiert. Die Fehlergrenzen für die registrierten Atome betragen: C: ±0,3 %, H: ±0,1 %, N: ±0,2 %.

### Beispiel 1: Ammonium-Tetracyanoborat, NH₄[B(CN)₄]

0.31 g (2.0 mmol) K[B(CN)₄] werden in 8 mL Wasser gelöst, danach mit einer Lösung von 0.20 g (1.1 mmol) (NH₄)₂[SiF₆] in 8 mL Wasser umgesetzt. Im Vakuum werden alle flüchtigen Bestandteile entfernt. Aus dem Rückstand wird NH₄[B(CN)₄] mit 10 mL CH₃CN extrahiert. Die organische Phase wird am Rotationsverdampfer eingeengt. Das Rohprodukt wird mit 10 mL CH₂Cl₂ gewaschen und im Vakuum getrocknet. Ausbeute 0.25 g (93%, 1.9 mmol).

Oberhalb von 300°C zersetzt sich laut DSC-Messungen das Salz.

### Beispiel 2: Trityl-Tetracyanoborat, [Ph₃C][B(CN)₄]

Es werden 500 mg (2.3 mmol) Ag[B(CN)₄] und 726 mg (2.3 mmol) (C₆H₅)₃CBr in wasserfreiem Acetonitril in einem 250 mL Glaskolben mit PTFE-Ventil (Young, London) zur Reaktion gebracht. Das Acetonitril wird nach 4 Std. im Vakuum entfernt und anschließend werden 100 mL Dichlormethan zugegeben. Die Suspension wird durch eine mit Celite® überschichtete Fritte in einen Schlenk-Kolben filtriert. Der Reaktionskolben wird zweimal mit Dichlormethan (20 mL und 10 mL) nachgespült. Unter reduziertem Druck wird die Lösung bis auf 10 mL eingeengt und nach Zugabe von 70 mL wasserfreiem Hexan fällt ein orange gefärbter Feststoff aus. Dieser wird über eine Schlenk-Fritte abfiltriert und mit weiteren 10 mL Hexan nachgewaschen. Das orange [Ph₃C][B(CN)₄] wird im Vakuum getrocknet und in einer Trockenbox gelagert. Die Ausbeute beträgt 408 mg (51%, 1.3 mmol).
¹H-NMR: δ = 7,73 ppm (m, 6H, o-H), δ = 7,94 ppm (m, 6H, *m*-H), δ = 8,31 ppm (tt, 3H, *p*-H); ¹³C{¹H}-NMR: δ = 122,7 ppm (q, 4C, CN), ¹*J*(¹¹B,¹³C) = 71,5 Hz, δ = 131,0 ppm (s, 6C, *m*-C), δ = 140,2 ppm (s, 3C, *i*-C), δ = 143,0 ppm (s, 6C, *o*-C), δ = 143,8 ppm (s, 3C, *p*-C), δ = 211,2 ppm (s, 1C, C⁺); ¹¹B-NMR: δ = -38,6 ppm, ¹*J*(¹¹B,¹³C) = 71,3 Hz; Lösungsmittel: CDCl₃ Referenzsubstanzen: ¹H- und ¹³C-NMR Lösungsmittelsignal (gegen TMS) und ¹¹B-NMR BF₃·Et₂O/CD₃CN als externer Standard

| Ergebnisse der Elementaranalyse [Ph₃C][B(CN)₄]: | | | |
|---|---|---|---|
| | C [%] | H [%] | N [%] |
| theoretisch | 77,12 | 4,22 | 15,64 |
| gefunden | 77,19 | 4,21 | 15,50 |

| | | | |
|---|---|---|---|
| [Ph₃C][B(CN)₄] schmilzt bei 158°C unter Zersetzung. | | | |

### Beispiel 3: [HNPhMe₂][B(CN)₄]

In 50 mL Wasser werden 1.50 g (9.7 mmol) K[B(CN)₄] gelöst. Unter Rühren werden zuerst 3 mL (36 mmol) konz. HCI-Lösung und anschließend 1.23 mL (9.7 mmol) *N,N*-Dimethylanilin zu der Lösung gegeben, woraufhin ein weißer Feststoff ausfällt. Mit Dichlormethan wird die Lösung zweimal extrahiert (100 mL und 30 mL), die organische Phase mit MgSO₄ getrocknet und das Dichlormethan im Vakuum entfernt. Es wird weißes [HNPhMe₂][B(CN)₄] erhalten, das durch Waschen mit Pentan gereinigt wird. Ausbeute 2.12 g (92%, 8.9 mmol).
¹H-NMR: δ = 3,23 ppm (s, 6H, CH₃), ¹Δ¹H(^{12/13}C) = -0,0023, ¹*J*(¹H,¹³C) = 145,48 Hz, δ = 7,64-7,58 ppm (m, 5H, C₆H₅); ¹³C{¹H}-NMR: δ = 47,8 ppm (s, 2C, CH₃), δ = 121,5 ppm (s, 2C, C₆H₅), δ = 123,2 ppm (s, 4C, CN), ¹*J*(¹¹B,¹³C) = 71,3 Hz, ¹Δ¹³C(^{10/11}B) = -0,0020 ppm, δ = 131,5 ppm (s, 2C, C₆H₅), δ = 131,6 ppm (s, 1C, C₆H₅), δ = 143,1 ppm (s, 1C, C₆H₅); ¹¹B-NMR: δ = -38,6 ppm, ¹*J*(¹¹B,¹³C) = 71,3 Hz; ¹⁵N-NMR: δ = -103,2 ppm (q, 4N, CN), ¹*J*(¹¹B,¹⁵N) = 0,73 Hz; Lösungsmittel: CD₃CN; Referenzsubstanzen: ¹H- und ¹³C-NMR Lösungsmittelsignal (gegen TMS), ¹¹B-NMR BF₃-Et₂O/CD₃CN als externer Standard und ¹⁵N-NMR 80 % CH₃NO₂ in CD₃CN als externer Standard.

| Ergebnisse der Elementaranalyse von [HNPhMe₂][B(CN)₄]: | | | |
|---|---|---|---|
| | C[%] | H [%] | N [%] |
| theoretisch | 60,80 | 5,10 | 29,54 |
| gefunden | 60,60 | 4,65 | 28,50 |

| | | | |
|---|---|---|---|
| [HNPhMe₂][B(CN)₄] schmilzt bei 101°C und zersetzt sich exotherm oberhalb von 246°C. | | | |

### Beispiel 4: Tetraethylammonium-Tetracyanoborat, [Et₄N][B(CN)₄]

In 300 mL Wasser werden 7 g (46 mmol) K[B(CN)₄] und in 130 mL Wasser 8.4 g (46 mmol) [Et₄N]Cl·H₂O gelöst. Beide Lösungen werden zusammengegeben, woraufhin ein weißer Feststoff ausfällt. Nach 30 Minuten Rühren werden 250 mL Dichlormethan zugegeben, in dem sich die ausgefallene Substanz löst. Die beiden Phasen werden getrennt und die organische Phase wird über MgSO₄ getrocknet. Am Rotationsverdampfer wird das Dichlormethan entfernt, der weiße Feststoff mehrmals mit Pentan gewaschen und anschließend im Vakuum getrocknet. Ausbeute 10.5 g (96%, 43 mmol).
¹H-NMR: δ = 1,22 ppm (tt, 12H, CH₃), ¹Δ¹H(^{12/13}C) = -0,0019 ppm, ¹*J*(¹H,¹³C) = 128,78 Hz, ³*J*(¹H,¹H) = 7,27 Hz; δ = 3,13 ppm (q, 8H, CH₂), ¹Δ¹H(^{12/13}C) = 0,0034 ppm, ¹*J*(¹H,¹³C) = 144,30 Hz, ²*J*(¹H,¹⁴N) = 1,89 Hz, ³*J*(¹H,¹H) = 7,28 Hz; ¹³C{¹H}-NMR: δ = 7,8 ppm (s, 4C, CH₃); δ = 53,2 ppm (t, 4C, CH₂), ¹*J*(¹³C,¹⁵N) = 3,1 Hz; δ = 123,3 ppm (q, 4C, CN), ¹Δ¹³C(^{10/11}B) = 0,0021 ppm, ¹*J*(¹¹B,¹³C) = 70,9 Hz; ¹¹B-NMR: δ = -38,6 ppm, ¹*J*(¹¹B,¹³C) = 71,2 Hz; Lösungsmittel: CD₃CN Referenzsubstanzen: ¹H- und ¹³C-NMR Lösungsmittelpeak (gegen TMS) und ¹¹B-NMR BF₃-Et₂O/CD₃CN als externer Standard.

| Ergebnisse der Elementaranalyse von [Et₄N][B(CN)₄]: | | | |
|---|---|---|---|
| | C [%] | H [%] | N [%] |
| theoretisch | 58.8 | 8.22 | 28.57 |
| gefunden | 58.5 | 8.18 | 28.22 |

| | | | |
|---|---|---|---|
| [Et₄N][B(CN)₄] schmilzt bei 230°C. Eine weitere reversible Phasenumwandlung findet bei einer Temperatur von 145°C statt. Oberhalb von 360°C zersetzt sich das Salz. | | | |

### Beispiel 5: 1-Butyl-3-methylimidazolium-Tetracyanoborat [C₈H₁₅N₂][B(CN)₄]

In 20 mL Wasser werden 0.35 g (2.3 mmol) K[B(CN)₄] gelöst. Unter Rühren werden 0.53 g (3.0 mmol) [C₈H₁₅N₂]Cl in 20 mL Wasser zugegeben. Mit Dichlormethan wird die Lösung zweimal extrahiert (30 mL und 20 mL), die organische Phase mit Wasser (20 mL) gewaschen, mit MgSO₄ getrocknet und anschließend das Dichlormethan im Vakuum entfernt. Ausbeute 0.50 g (87%, 2.0 mmol).

| Ergebnisse der Elementaranalyse von [C₈H₁₅N₂][B(CN)₄]: | | | |
|---|---|---|---|
| | C [%] | H [%] | N [%] |
| theoretisch | 56,70 | 5,95 | 33,07 |
| gefunden | 56,24 | 6,13 | 32,99 |

| | | | |
|---|---|---|---|
| [C₈H₁₅N₂][B(CN)₄] schmilzt unter -50°C und zersetzt sich endotherm oberhalb von 410°C. | | | |

### Beispiel 6: 1-Ethyl-3-methylimidazolium-Tetracyanoborat [C₆H₁₁N₂][B(CN)₄]

[C₆H₁₁N₂][B(CN)₄] wird analog zu [C₈H₁₅N₂][B(CN)₄] mit der gleichen Ausbeute hergestellt.

| Ergebnisse der Elementaranalyse von [C₆H₁₁N₂][B(CN)₄]: | | | |
|---|---|---|---|
| | C [%] | H [%] | N [%] |
| theoretisch | 53,13 | 4,90 | 37,18 |
| gefunden | 52,79 | 4,97 | 37,12 |

| | | | |
|---|---|---|---|
| [C₆H₁₁N₂][B(CN)₄] schmilzt unter -50°C und zersetzt sich endotherm oberhalb von 420°C. | | | |

### Beispiel 7: p-Methyl-butylpyridinium-Tetracyanoborat [C₁₀H₁₆N][B(CN)₄]

[C₁₀H₁₆N][B(CN)₄] wird analog zu [C₈H₁₅N₂][B(CN)₄] mit der gleichen Ausbeute hergestellt.

| Ergebnisse der Elementaranalyse von [C₁₀H₁₆N][B(CN)₄]: | | | |
|---|---|---|---|
| | C [%] | H [%] | N [%] |
| theoretisch | 63,42 | 6,08 | 26,42 |
| gefunden | 62,81 | 6,13 | 26,70 |

| | | | |
|---|---|---|---|
| [C₁₀H₁₆N][B(CN)₄] erstarrt bei -25°C, schmilzt bei 42°C und zersetzt sich endotherm oberhalb von 390°C. | | | |

### Beispiel 8: 1-Ethyl-3-methylimidazolium-Tricyanofluoroborat [C₆H₁₁N₂][BF(CN)₃]

[C₆H₁₁N₂][BF(CN)₃] wird analog zu [C₈H₁₅N₂][B(CN)₄] mit der gleichen Ausbeute hergestellt.

| Ergebnisse der Elementaranalyse von [C₆H₁₁N₂][BF(CN)₃]: | | | |
|---|---|---|---|
| | C[%] | H[%] | N[%] |
| theoretisch | 49.35 | 5.06 | 31.98 |
| gefunden | 48.52 | 4.84 | 31.20 |

| | | | |
|---|---|---|---|
| [C₆H₁₁N₂][BF(CN)₃] ist bei Raumtemperatur flüssig. | | | |

### Beispiel 9: 1-Butyl-3-methylimidazolium-Tricyanofluoroborat [C₈H₁₅N₂][BF(CN)₃]

[C₈H₁₅N₂][BF(CN)₃] wird analog zu [C₈H₁₅N₂][B(CN)₄] mit der gleichen Ausbeute hergestellt.

| Ergebnisse der Elementaranalyse von [C₈H₁₅N₂][BF(CN)₃]: | | | |
|---|---|---|---|
| | C[%] | H[%] | N[%] |
| theoretisch | 53.47 | 6.12 | 28.34 |
| gefunden | 54.06 | 6.09 | 28.68 |

| | | | |
|---|---|---|---|
| [C₈H₁₅N₂][BF(CN)₃] schmilzt unter -50°C und zersetzt sich exotherm oberhalb von 300°C. | | | |

### Beispiel 10: p-Methyl-butylpyridinium-Tricyanofluorobortat [C₁₀H₁₆N][BF(CN)₃]

[C₁₀H₁₆N][BF(CN)₃] wird analog zu [C₈H₁₅N₂][B(CN)₄] mit der gleichen Ausbeute hergestellt.

| Ergebnisse der Elementaranalyse von [C₁₀H₁₆N][BF(CN)₃]: | | | |
|---|---|---|---|
| | C [%] | H [%] | N[%] |
| theoretisch | 60.50 | 6.25 | 21.71 |
| gefunden | 61.13 | 5.51 | 22.35 |

| | | | |
|---|---|---|---|
| [C₁₀H₁₆N][BF(CN)₃] schmilzt unter -50°C und zersetzt sich exotherm oberhalb von 260°C. | | | |

### Beispiel 11: 1-Ethyl-3-methylimidazolium-Dicyanodifluoroborat [C₆H₁₁N₂][BF₂(CN)₂]

[C₆H₁₁N₂][BF₂(CN)₂] wird analog zu [C₈H₁₅N₂][B(CN)₄] mit der gleichen Ausbeute hergestellt.

| Ergebnisse der Elementaranalyse von [C₆H₁₁N₂][BF₂(CN)₂]: | | | |
|---|---|---|---|
| | C [%] | H [%] | N [%] |
| theoretisch | 45.32 | 5.23 | 26.43 |
| gefunden | 45.14 | 5.14 | 26.28 |

| | | | |
|---|---|---|---|
| [C₆H₁₁N₂][BF₂(CN)₂] schmilzt unter -50°C und zersetzt sich exotherm oberhalb von 200°C. | | | |

### Beispiel 12: 1-Butyl-3-methylimidazolium-Dicyanodifluoroborat [C₈H₁₅N₂][BF₂(CN)₂]

[C₈H₁₅N₂][BF₂(CN)₂] wird analog zu [C₈H₁₅N₂][B(CN)₄] mit der gleichen Ausbeute hergestellt.

| Ergebnisse der Elementaranalyse von [C₈H₁₅N₂][BF₂(CN)₂]: | | | |
|---|---|---|---|
| | C [%] | H [%] | N [%] |
| Theoretisch | 50.03 | 6.30 | 23.34 |
| Gefunden | 50.20 | 6.31 | 23.42 |

| | | | |
|---|---|---|---|
| [C₈H₁₅N₂][BF₂(CN)₂] schmilzt unter -50°C und zersetzt sich exotherm oberhalb von 210°C. | | | |

### Beispiel 13: p-Methyl-butylpyridinium-Dicyanodifluoroborat [C₁₀H₁₆N][BF₂(CN)₂]

[C₁₀H₁₆N][BF₂(CN)₂] wird analog zu [C₈H₁₅N₂][B(CN)₄] mit der gleichen Ausbeute hergestellt.

| Ergebnisse der Elementaranalyse von [C₁₀H₁₆N][BF₂(CN)₂]: | | | |
|---|---|---|---|
| | C [%] | H [%] | N [%] |
| theoretisch | 57.40 | 6.42 | 16.74 |
| gefunden | 57.70 | 6.20 | 16.95 |

| | | | |
|---|---|---|---|
| [C₁₀H₁₆N][BF₂(CN)₂] schmilzt unter -50°C und zersetzt sich exotherm oberhalb von 190°C. | | | |

## Patentansprüche

1. Verwendung eines Salzes der allgemeinen Formel (3)
Kt⁺ [BFₙ(CN)₄₋ₙ]⁻ (3),
wobei n = 0, 1, 2 oder 3 und Kt⁺ ein organisches Kation ist
als ionische Flüssigkeit oder als nicht-wässriger Elektrolyt oder als Phasentransferkatalysator oder als Zwischenprodukt für die Synthese von Flüssigkristallverbindungen oder Arznei- oder Pflanzenschutzmitteln.

2. Salz der allgemeinen Formel (3)
Kt⁺ [BFₙ(CN)₄₋ₙ]⁻ (3)
wobei n = 0, 1, 2 oder 3 und Kt⁺ ein organisches Kation ist, mit der Maßgabe, dass für n=0 das Kation Kt⁺ nicht [N(C₄H₉)₄]⁺ ist.

3. Salz nach Anspruch 2,
**dadurch gekennzeichnet, dass** das organische Kation Kt⁺ ausgewählt ist aus der Gruppe 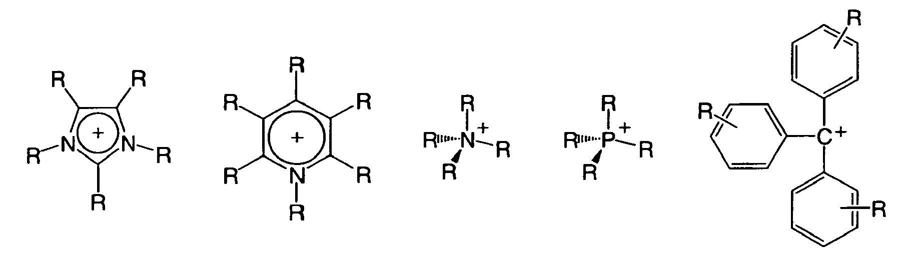 mit
R = H mit der Maßgabe, dass mindestens ein R am Heteroatom von H verschieden ist,
geradkettiges oder verzweigtes Alkyl mit 1-20 Kohlenstoffatomen
geradkettiges oder verzweigtes Alkenyl mit 2-20 Kohlenstoffatomen und einer oder mehreren Doppelbindungen
geradkettiges oder verzweigtes Alkinyl mit 2-20 Kohlenstoffatomen und einer oder mehreren Dreifachbindungen
gesättigtes, teilweise oder vollständig ungesättigtes Cycloalkyl mit 3-7 Kohlenstoffatomen
Halogen, insbesondere Fluor oder Chlor, mit der Maßgabe, dass keine Halogen-Heteroatom-Bindung vorliegt,
-NO₂ mit der Maßgabe, dass keine Bindung zu einem positiv geladenen Heteroatom vorliegt und mindestens ein R von NO₂ verschieden ist,
-CN mit der Maßgabe, dass keine Bindung zu einem positiv geladenen Heteroatom vorliegt und mindestens ein R von CN verschieden ist,
wobei die R jeweils gleich oder verschieden sind,
wobei die R paarweise durch Einfach- oder Doppelbindung miteinander verbunden sein können,
wobei ein oder mehrere R teilweise oder vollständig mit Halogenen, insbesondere -F und/oder -Cl, oder teilweise mit -CN oder -NO₂, substituiert sein können, mit der Maßgabe, dass nicht alle R vollständig halogeniert sind,
und wobei ein oder zwei Kohlenstoffatome des R durch Heteroatome und/oder Atomgruppierungen ausgewählt aus der Gruppe -O-, -C(O)-, C(O)O-, -S-, -S(O)-, -SO₂-, -S(O)₂O-, -N=, -P=, -NR'-, -PR'-, -P(O)(OR')-, -P(O)(OR')O-, -P(O)(NR'R')-, -P(O)(NR'R')O-, -P(O)(NR'R')NR'-, -S(O)NR'- und -S(O)₂NR'- mit R' = H, nicht, teilweise oder perfluoriertes C₁- bis C₆-Alkyl oder nicht, teilweise oder perfluoriertes Phenyl,
ersetzt sein können.

4. Salz nach Anspruch 2 oder 3,
**dadurch gekennzeichnet, dass** das organische Kation Kt⁺ ausgewählt ist aus der Gruppe 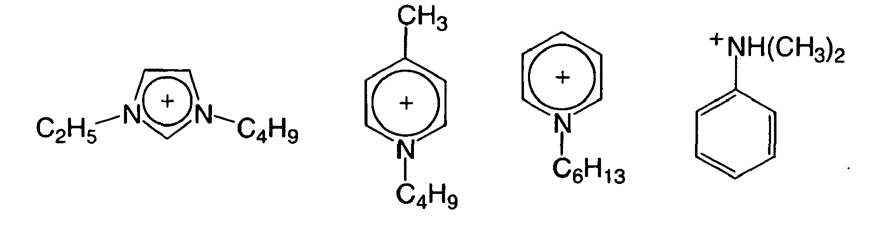 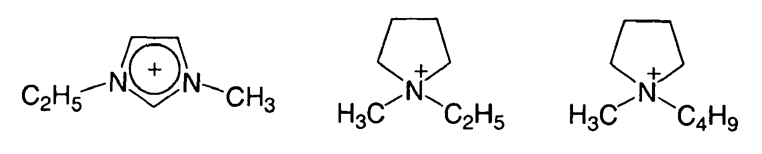 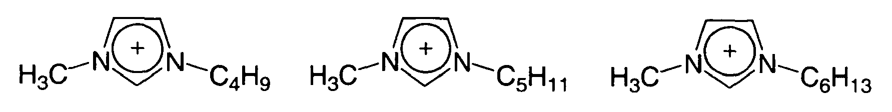 N(C₂H₅)₄⁺ P(C₂H₅)₄⁺ P(C₄H₉)₄⁺ P(C₆H₁₃)₃(C₁₄H₂₉)⁺

5. Verfahren zur Herstellung eines Salzes der allgemeinen Formel (3)
Kt⁺ [BFₙ(CN)₄₋ₙ]⁻ (3),
mit der Maßgabe, dass für n=0 das Kation Kt⁺ nicht [N(C₄H₉)₄]⁺ ist,
**dadurch gekennzeichnet, dass** ein Alkalimetallcyanoborat der allgemeinen Formel M⁺ [BFₙ(CN)₄₋ₙ]⁻, wobei n = 0, 1, 2 oder 3 und M ausgewählt ist aus der Gruppe Li, Na, K, Rb und Cs,
mit Kt⁺ X⁻ umgesetzt wird,
wobei X ein Halogen ausgewählt aus Cl, Br und I und Kt⁺ ein organisches Kation ist, mit der Maßgabe, dass das Kation Kt⁺ für n=0 nicht [N(C₄H₉)₄]⁺ ist.

## Claims

1. Use of a salt of the general formula (3)
Kt⁺ [BFₙ(CN)₄₋ₙ]⁻ (3),
where n = 0, 1, 2 or 3, and Kt⁺ is an organic cation,
as ionic liquid or as non-aqueous electrolyte or as phase-transfer catalyst or as intermediate for the synthesis of liquid-crystal compounds or medicaments or crop-protection agents.

2. Salt of the general formula (3)
Kt⁺ [BFₙ(CN)₄₋ₙ]⁻ (3),
where n = 0, 1, 2 or 3, and Kt⁺ is an organic cation, with the proviso that for n = 0 the cation Kt⁺ is not [N(C₄H₉)₄]⁺.

3. Salt according to Claim 2,
**characterised in that** the organic cation Kt⁺ is selected from the group 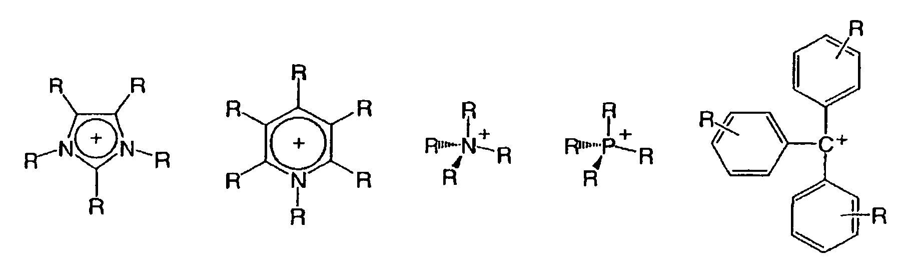 where
R = H, with the proviso that at least one R on the heteroatom is different from H,
straight-chain or branched alkyl having 1-20 carbon atoms
straight-chain or branched alkenyl having 2-20 carbon atoms and one or more double bonds
straight-chain or branched alkynyl having 2-20 carbon atoms and one or more triple bonds
saturated, partially or fully unsaturated cycloalkyl having 3-7 carbon atoms
halogen, in particular fluorine or chlorine, with the proviso that no halogen-heteroatom bond is present,
-NO₂, with the proviso that no bond to a positively charged heteroatom is present, and at least one R is different from NO₂,
-CN, with the proviso that no bond to a positively charged heteroatom is present, and at least one R is different from CN,
where the R are in each case identical or different,
where the R may be bonded to one another in pairs by single or double bond,
where one or more R may be partially or fully substituted by halogens, in particular -F and/or -Cl, or partially by -CN or -NO₂, with the proviso that not all R are fully halogenated,
and where one or two carbon atoms of the R may be replaced by heteroatoms and/or atom groups selected from the group -O-, -C(O)-, -C(O)O-, -S-, -S(O)-, -SO₂-, -S(O)₂O-, -N=, -P=, -NR'-, -PR'-, -P(O)(OR')-, -P(O)(OR')O-, -P(O)(NR'R')-, -P(O)(NR'R')O-, -P(O)(NR'R')NR'-, -S(O)NR'- and -S(O)₂NR'-, where R' = H, non-, partially or perfluorinated C₁- to C₆-alkyl or non-, partially or perfluorinated phenyl.

4. Salt according to Claim 2 or 3,
**characterised in that** the organic cation Kt⁺ is selected from the group 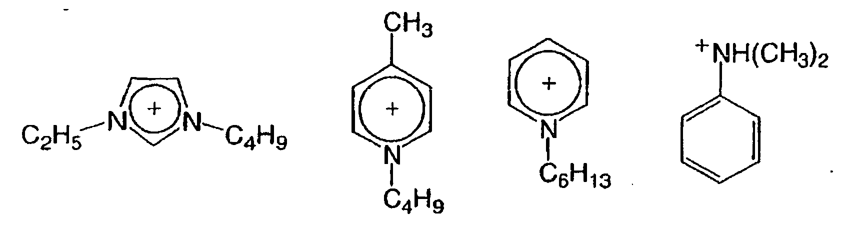 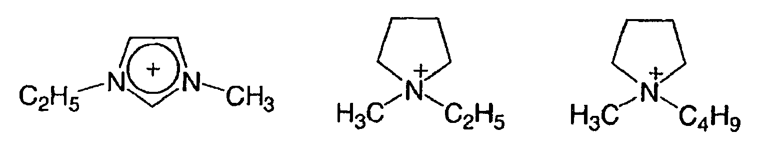 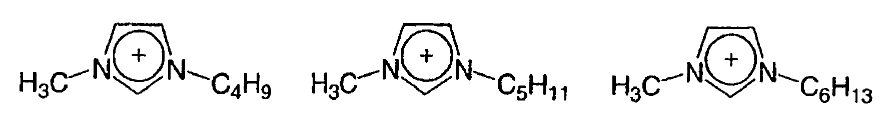 N(C₂H₅)₄⁺ P(C₂H₅)₄⁺ P(C₄H₉)₄⁺ P(C₆H₁₃)₃(C₁₄H₂₉)⁺

5. Process for the preparation of a salt of the general formula (3)
Kt⁺ [BFₙ(CN)₄₋ₙ]⁻ (3),
with the proviso that for n=0 the cation Kt⁺ is not [N(C₄H₉)₄]⁺,
**characterised in that** an alkali metal cyanoborate of the general formula M⁺ [BFₙ(CN)₄₋ₙ]⁻, where n = 0, 1, 2 or 3, and M is selected from the group Li, Na, K, Rb and Cs,
is reacted with Kt⁺ X⁻,
where X is a halogen selected from Cl, Br and I, and Kt⁺ is an organic cation, with the proviso that for n = 0 the cation Kt⁺ is not [N(C₄H₉)₄]⁺.

## Revendications

1. Utilisation d'un sel de la formule générale (3)
Kt⁺ [BFₙ(CN)₄₋ₙ]⁻ (3),
dans laquelle n = 0, 1, 2 ou 3, et Kt⁺ est un cation organique,
en tant que liquide ionique ou en tant qu'électrolyte non aqueux ou en tant que catalyseur de transfert de phase ou en tant que produit intermédiaire pour la synthèse de composés de cristal liquide ou de médicaments ou d'agents de protection des cultures.

2. Sel de la formule générale (3)
Kt⁺ [BFₙ(CN)₄₋ₙ]⁻ (3),
dans laquelle n = 0, 1, 2 ou 3, et Kt⁺ est un cation organique, étant entendu que pour n = 0, le cation Kt⁺ n'est pas [N(C₄H₉)₄]⁺.

3. Sel selon la revendication 2,
**caractérisé en ce que** le cation organique Kt⁺ est choisi parmi le groupe 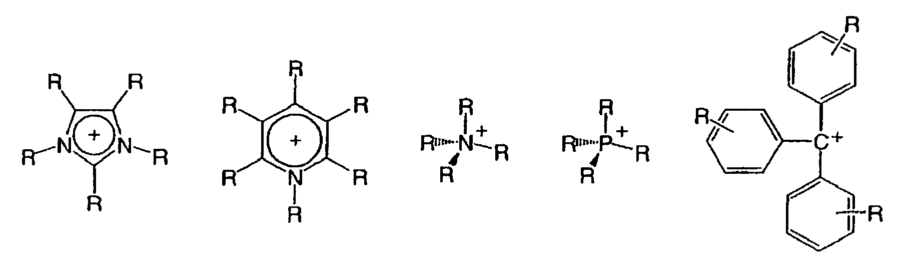 où
R = H, étant entendu qu'au moins un R sur l'hétéroatome est différent de H,
alkyle en chaîne droite ou ramifié comportant 1-20 atomes de carbone
alkényle en chaîne droite ou ramifié comportant 1-20 atomes de carbone et une ou plusieurs liaisons doubles
alkynyle en chaîne droite ou ramifié comportant 1-20 atomes de carbone et une ou plusieurs liaisons triples
cycloalkyle saturé, partiellement ou complètement insaturé comportant 3-7 atomes de carbone
halogène, en particulier fluor ou chlore, étant entendu que aucune liaison halogène-hétéroatome n'est présente,
-NO₂, étant entendu qu'aucune liaison sur un hétéroatome positivement chargé n'est présente, et au moins un R est différent de NO₂,
-CN, étant entendu qu'aucune liaison sur un hétéroatome positivement chargé n'est présente, et au moins un R est différent de CN,
où les R sont dans chaque cas identiques ou différents,
où les R peuvent être liés les uns aux autres selon des paires par liaison simple ou double,
où un ou plusieurs R peuvent être partiellement ou complètement substitués par des halogènes, en particulier -F et/ou -CI, ou partiellement par -CN ou -NO₂, étant entendu que les R ne sont pas tous complètement halogénés,
et où un ou deux atomes de carbone des R peuvent être remplacés par des hétéroatomes et/ou des groupes d'atomes choisis parmi le groupe -O-, -C(O)-, -C(O)O-, -S-, -S(O)-, -SO₂-, -S(O)₂O-, -N=, -P=, -NR'-, -PR'-, -P(O)(OR')-, -P(O)(OR')O-, -P(O)(NR'R')-, -P(O)(NR'R')O-, -P(O)(NR'R')NR'-, -S(O)NR'- et -S(O)₂NR'-,
où R' = H, C₁- à C₆-alkyle non perfluorés, partiellement perfluorés ou perfluorés ou phényle non perfluoré, partiellement perfluoré ou perfluoré.

4. Sel selon la revendication 2 ou 3,
**caractérisé en ce que** le cation organique Kt⁺ est choisi parmi le groupe 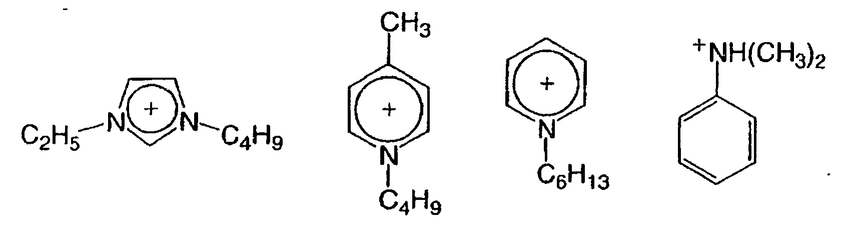 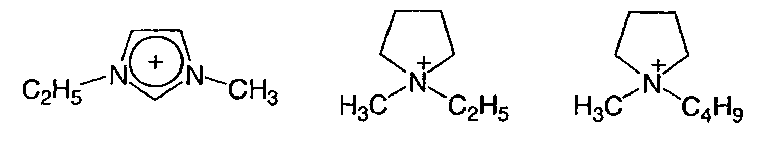 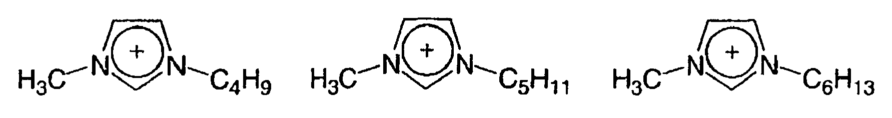 N(C₂H₅)₄⁺ P(C₂H₅)₄⁺ P(C₄H₉)₄⁺ P(C₆H₁₃)₃(C₁₄H₂₉)⁺

5. Procédé pour la préparation d'un sel de la formule générale (3)
Kt⁺ [BFₙ(CN)₄₋ₙ]⁻ (3),
étant entendu que pour n=0, le cation Kt⁺ n'est pas [N(C₄H₉)₄]⁺,
**caractérisé en ce qu**'un cyanoborate de métal alcalin de la formule générale M⁺ [BFₙ(CN)₄₋ₙ]⁻, où n = 0, 1, 2 ou 3, et M est choisi parmi le groupe Li, Na, K, Rb et Cs,
est amené à réagir avec Kt⁺ X⁻,
où X est un halogène choisi parmi CI, Br et I, et Kt⁺ est un cation organique, étant entendu que pour n = 0, le cation Kt⁺ n'est pas [N(C₄H₉)₄]⁺.
